# EUROPEAN PATENT APPLICATION

(11) **EP 4 173 613 A1**
(43) Date of publication of application: **03.05.2023**
(21) Application number: 21205288.0
(22) Date of filing: 28.10.2021
(51) Int. Cl.: A61K 9/00, A61K 9/14, A61K 31/12

(54) **DERMAL APPLICATION OF CHEMICAL COMPOUNDS FORMULATED AS NANOPARTICLES**

(71) Applicant: PHILIPPS-UNIVERSITÄT MARBURG, 35037 Marburg (DE)
(72) Inventor: Keck, Cornelia, 14548 Schwielowsee (DE); Pelikh, Olga, 79539 Lörrach (DE)
(74) Representative: Durm Patentanwälte PartG mbB

(57) **Abstract**

The application relates to a method for transporting poorly soluble, very poorly soluble and practically insoluble chemical compounds formulated as nanoparticles into and through the intact skin, as well as for controlled releasing of said chemical compound formulated as nanoparticles thereafter, wherein said chemical compound formulated as nanoparticles are applied to the intact skin and treated thereafter with a means for treating the intact skin selected from microneedles, dermaroller and laser so that the chemical compound formulated as nanoparticles are transported into and through the intact skin. Furthermore, the use of chemical compounds formulated as nanoparticles as well as a method for operating microneedles, a dermaroller and laser in such a method is disclosed.

## Description

The present invention relates to a method for transporting poorly soluble, very poorly soluble and practically insoluble chemical compounds formulated as nanoparticles into and through the intact skin as well as to controlled releasing them. The present invention further relates to the use of chemical compounds formulated as nanoparticles in such a method as well as a method for operating a means for treating the intact skin in such a method.

After dermal application, an active ingredient only passes through the skin if it is available dissolved at the site of absorption. If an active ingredient is poorly soluble, special formulation strategies are needed to increase the solubility of the active ingredients accordingly. In the field of dermal formulations, formulations that increase the dermal penetration of poorly soluble active ingredients include, for example, microemulsions, nanoemulsions, lipidnanoparticles, liposomes or nanocrystals. It is also known that microneedles (microneedle patches, lasers, dermarollers) can temporarily open the skin barrier and thus improve the penetration of dissolved active ingredients into the skin.

To date, three strategies are available for improving the penetration of poorly soluble active ingredients. The first option is to improve solubility through galenic measures, the second is the targeted transport of particles into hair follicles, and the third method is the production of drug-loaded microneedles. For the first strategy, nanocrystals are best suited, as they consist of 100% active ingredient, thus can be considered as an effective carrier system. Nanocrystals are also best suited for hair follicle targeting, as they consist of 100% active ingredient, unlike other carriers. The disadvantage of hair follicle targeting is that only very small amounts of active ingredient or particle volume can be transported into the hair follicles, which can be explained by the small volume of the hair follicles (a few picoliters) and the general number of hairs on the skin. The third method transports active substances extremely effectively into the skin - however, only extremely small amounts of active substance can be loaded into the microneedles, so this method can only be used for highly potent substances (i.e. very low doses in the ng-pg range are sufficient for effective therapy). In summary it can be said that up to now no method is available to transport poorly soluble active substances effectively, quickly, inexpensively, in large quantities, into and through the skin without medical personnel.

Therefore, the technical problem underlying the present invention is to transport poorly soluble, very poorly soluble and practically insoluble chemical compounds formulated as nanoparticles effectively, quickly, inexpensively, in large quantities, into and through the intact skin without medical personnel.

This object has been solved by the subject-matter of the independent claims. Preferred embodiments are defined in the dependent claims.

According to the present invention, there is provided a method for transporting poorly soluble, very poorly soluble and practically insoluble chemical compounds formulated as nanoparticles into and through the intact skin, as well as for controlled releasing of said chemical compounds formulated as nanoparticles (for the sake of simplicity hereinafter it is referred to as "nanoparticles")thereafter, wherein said nanoparticles are applied to the intact skin and treated thereafter with a means for treating the intact skin selected from microneedles, for example microneedle rollers or microneedle patches, dermaroller, and laser, for example by massaging, so that the nanoparticles are transported into and through the intact skin, wherein furthermore optionally a viscosity-changing substance is used, with which a depot in the intact skin is provided, in which the nanoparticles are embedded so that the releasing of the nanoparticles is controlled.

The intact skin as used according to the present invention contains the epidermis, the dermis and the hypoderm. Intact skin does not mean healthy skin, but it includes as well damaged skin caused by diseases like neurodermatitis or psoriasis, or otherwise damaged skin for examples damaged by an insect bite as long as it contains the above three layers.

The skilled person knows materials and methods for formulating chemical compounds into nanoparticles. The nanoparticles can be nanocrystals, lipidnanoparticles or liposomes. The term "nano-" as used herein is understood as commonly used in the field of pharmacy.

As mentioned above, as means for transporting the nanoparticles through the intact skin, microneedles, dermaroller or laser can be applied. In the following, the invention is exemplified by the dermaroller, but it is explicitly pointed out that microneedles and laser can be applied in the same manner.

The skin is the outer protective shield of the body and protects it from the penetration of undesirable substances. However, the aim of pharmacy or cosmetics is to transport active ingredients specifically into the skin or through the skin into the body. For this purpose, the protective shield of the body must be overcome. The present invention addresses this issue. With the invention, it is possible to transport substances, which normally do not penetrate the intact skin or penetrate it only to a very small extent, in a targeted manner, in large quantities and without the assistance of medical personnel transdermally, i.e. through the intact skin, into deeper skin layers and to "deposit" them there over a longer period of time. The new method is universally applicable for poorly soluble, very poorly soluble and practically insoluble substances. The method is comparatively inexpensive and does not require expensive and complicated equipment or medical professionals. Formulation development is much simpler than was necessary for previous formulations for dermal or transdermal application. Therefore, this method finds wide application in pharmacy (including veterinary medicine) and cosmetics whenever it is intended to penetrate an active substance through the intact skin.

Completely unexpectedly and surprisingly, it was found experimentally that the application of nanoparticles and a subsequent massage using in particular a dermaroller allows the nanoparticles to effectively penetrate the intact skin. There they form a depot. If larger active ingredient particles are used instead of nanoparticles, penetration is very low. Without dermaroller, penetration is comparatively low - regardless of whether micro- or submicron particles are used.

The nanoparticles employed according to the present invention may be poorly soluble, very poorly soluble or practically insoluble; poorly soluble means 100 parts to 1000 parts, very poorly soluble means 1 000 parts to 10 000 parts and practically insoluble means more than 10 000 parts volume solvent are required to dissolve 1 mass part substance at 15 °C to 25 °C.

In one embodiment, the nanoparticles have a particle size of less than or equal to 1000 nm, for example 600 to 700 nm, wherein it is pointed out that the nanoparticles should be as small as possible. The particle size can be determined by dynamic light scattering, laser diffraction or microscopic methods.

In one embodiment, the used nanoparticles are suitable for parenteral administration.

In a further embodiment, the depot in the intact skin is provided by a gel cushion created in the intact skin, in which the nanoparticles are embedded. The gel cushion can be prepared beforehand or by applying the nanoparticles together with a liquid gelling agent to the intact skin and treating both with in particular the dermaroller so that the nanoparticles and the liquid gelling agent are transported into and through the intact skin and the liquid gelling agent gels after transport through the intact skin embedding the nanoparticles.

In one embodiment, the gelling occurs after transport into and through the intact skin due to a change of a pH-value, due to a change in temperature, and/or due to thixotropic properties of the liquid gelling agent.

The gelling agent can be selected from the group of hyaluronic acids, collagens, dextrans, PVP (polyvinylpyrrolidon), cellulose derivatives capable of forming a gel, gummi arabicum , gelatin and poloxamers, for example poloxamer 407, poloxamer 188, xanthan gum, carrageenan, polyacrylate.

To be more specifically, a modulable depot effect is provided according to the present invention. The nanoparticles penetrate into the intact skin and remain therein for example in deeper skin areas where they can provide a local effect or from where they are delivered systemically. The deeper skin area can be 0,25 mm, 0,5 mm, 1,0 mm, 2,0 mm and/or 3,0 mm under the outer surface of the skin. The depot effect in the intact skin can be modulated by the addition of viscosity-changing substances. There is created a "gel cushion" in the intact skin in which the nanoparticles (and additionally possibly other dissolved molecules) are embedded. The gel can be prepared in advance in the intact skin or the formulation is a liquid containing the nanoparticles and gels after application (e.g. by changing the pH in the intact skin (approx. 7.2) or the temperature (> room temperature) or due to thixotropic properties of the gel former). By varying the concentration of the gel former, more or less "solid" gel pads can be formed, wherein the active ingredient transport (occurs by diffusion) can then take place correspondingly faster or slower - i.e. a modulable depot effect is provided. Suitable gel formers that are of interest here are: Hyaluronic acids, collagens (especially interesting for cosmetics - would be a so to say "2in1" effect), dextranes, PVP, cellulose derivatives, gummi arabicum, gelatine (especially for pharma), poloxamers, for example poloxamer 407 and poloxamer 188, xanthan gum, carrageenan, polyacrylate, which forms gels when the temperature is increased (> 30 °C - we have it in the intact skin) - before that it is thin.

The modulable depot effect is advantageous because in principle that is exactly what is actually needed in reality: controllable kinetics, not just a lot going in at once no matter how, but exactly as much and for as long as it is needed at a particular point.

In one embodiment, the nanocrystal is composed of an active ingredient selected from the group consisting of nanoparticles being effective as analgesics, hormones, vaccines, hair growth agents, disinfectants, antiparasitics, fungicides, antibiotics, antifungals, antihistamines, local anaesthetics, bleaching agents and oxidizing agents (for tattoo removal), antibodies, DNA agents, RNA agents cells (regenerative and individualized therapies), anti-aging, antioxidants, pigments (permanent cosmetics), hair growth agents, anti-acne, hyaluronic acid depots, and disinfectants.

Subject-matter of the present invention are further poorly soluble, very poorly soluble and practically insoluble nanoparticles for use in transporting them into and through the intact skin, as well as for controlled releasing of said nanoparticles thereafter, wherein said nanoparticles are applied to the intact skin and treated thereafter with a means for treating the intact skin selected from the group consisting of microneedles, dermaroller and laser so that the nanoparticles are transported into and through the intact skin, wherein furthermore optionally viscosity-changing substances can be used, with which a depot in the intact skin is provided, in which the nanoparticles are embedded so that the releasing of the nanoparticles is controlled.

As to the details for the transporting through the intact skin and for the controlled releasing, it is referred to the above detailed explanations in their entirety.

In one embodiment, the nanoparticles are used for the treatment of pain, hair loss, hair disease, skin disorders, nail disorders, parasite infections, bacterial infections, fungi infections, treatment of addiction (smoking, THC), hormone disorders (including diabetes), blood pressure adjustment, heart rate adjustment, cancer treatment, treatment of mood, modulation of weight, modulation of appetite, supplementation of vitamins, minerals, trace elements, lung diseases, allergy treatment, prevention of pain, prevention of hair loss, prevention of oxidative stress and diseases/conditions related to oxidative stress. Prevention (including vaccination) of infections (bacteria, virus, fungi, parasites) acne, itching, rosacea, modulation of skin appearance.

A further subject-matter of the present invention is a method of operating a means for treating the intact skin selected from the group consisting of microneedles, dermaroller and laser for transporting poorly soluble, very poorly soluble and practically insoluble nanoparticles into and through the intact skin, as well as for controlled releasing of said nanoparticles thereafter, wherein said nanoparticles are applied to the intact skin and treated thereafter with a dermaroller so that the nanoparticles are transported into and through the intact skin, wherein furthermore optionally a viscosity-changing substance is used, with which a depot in the intact skin is provided, in which the nanoparticles are embedded so that the realising of the nanoparticles is controlled.

Regarding the details of the use, it is referred to the above detailed description of the method in its entirety. Furthermore, it is pointed out that the laser and microneedles and dermarollers can be purchased commercially.

Moreover, the present invention concerns the use of microneedles, dermaroller and laser in the above defined method.

More specifically, the present invention makes it possible to transport for example poorly soluble active pharmaceutical ingredients into the body formulated as nanocrystals, lipidnanoparticles, liposomes in large quantities. Possible applications are the following:
- painkillers
- hormones
- vaccines
- hair restorer
- disinfectants
- antiparasitics, fungicides
- antibiotics
- antimycotics,
- antihistamines
- local anaesthetics
- bleaching and oxidizing agents (for tattoo removal)
- antibodies
- DNA agents, RNA agents
- cells (regenerative and individualized therapies).

The invention also makes it possible to transport cosmetic active ingredients into the body in large quantities. Possible applications are as follows:
- anti-aging, antioxidants
- pigments (permanent cosmetics)
- hair restorer
- anti-acne agents
- hyaluronic acid depots
- disinfectants
- antimycotics
- antihistamines

Therefore, the present invention can be used in the pharmaceutical industry, for example for administering drugs for the immediate treatment of insect bites and stings. The direct application of active ingredients to the puncture or bite can immediately stop the spread of pathogens.

Further possible applications are:
- Local antibiotic application after tick bite
- Prevention of Lyme disease.
- Local antibiotics/virostatics after bug bites or mosquito bites (possibility of prevention of many tropical diseases with severe and often fatal courses)
- Local application of antihistamines or corticosteroids
- reduction of allergies, pain and itching after wasp, bee or mosquito bites.

Other possible applications in pharmacy or medicine are:
- Application of vaccines
- Application of proteins (e.g. insulin) - here enormous advantage compared to conventional therapies (injections) - because more comfortable and safer
- Application of painkillers - e.g. joint pain - if necessary, a long-term systemic drug application can be reduced or suspended - since long-term painkiller therapy often leads to kidney damage and stomach problems, a better therapy can be made possible here.
- Transdermal application of hormones (improved hormone replacement therapy)
- All dermal applications where previous formulation strategies could not transport sufficient amounts of active ingredients into the skin (e.g. therapy of chronic skin diseases, wounds, scars, skin tumors, etc.).

The invention can also be used in cosmetics, for example for enhanced penetration of cosmetic active ingredients - e.g. antioxidants, proteins - collagen, hyaluronic acid, Botox derivatives or lipids (triglycerides, ceramides, sphingosines, free fatty acids, cholesterol and derivatives, phospholipids) and other substances (minerals, small organic molecules - e.g. sugars, urea).

The invention is further exemplified by the following examples in connection with the figures. It is explicitly pointed out that the examples and figures shall not be considered to limit the invention thereto, but they are intended for illustrative purpose only.
Figure 1 shows the digital image analysis regarding the total amount penetrated (Fig. 1A) and the mean penetration depth (Fig. 1B) obtained in the example.
Figure 2 shows the comparison of the relative amount penetrated (Fig. 2A) and the relative penetration depth (Fig. 2B) obtained in the example.

### Example:

The following materials have been used in the example: curcumin; microcrystals bulk material particle size approx. 10 µm; nanocrystals nanosuspension particle size approx. 500 nm; intact skin from fresh porcine skin; impaired skin from fresh porcine skin, tape stripped 30 times, mimics atopic dermatitis; manipulated skin from fresh porcine skin, treated with dermaroller after application of formulation.

### Application protocol:

Pig ear skin was used for the experiments:
Ear side: dorsal side
Area: 1.5 x 1.5 cm²
Application volume: 50 µL
Application method: 1 minute finger massage
Application of control formulation: 1 minute finger massage.
Application with tape stripping: the marked skin area was tape stripped 30 times. The formulation was then applied and massaged in for 1 minute.
Application with dermaroller: the formulation was applied to the marked skin area and massaged in for 1 minute. Then the dermaroller was applied: it was "run" over the formulation for 1 minute in 4 directions: across as well as cross.
Penetration time: 4 hours at 32 °C
Afterwards: wash off formulation, punch out the pre-treated area (diameter 15 mm), embed obtained biopsies, freeze, cut.

### Data analysis:

determination of dermal penetration efficacy:
visualization of dermal penetration efficacy with epifluorescence microscopy *)
quantification of dermal penetration efficacy with digital image analysis *)

*) according to Pelikh O, Pinnapireddy S, R, Keck C, M: Dermal Penetration Analysis of Curcumin in an ex vivo Porcine Ear Model Using Epifluorescence Microscopy and Digital Image Processing. Skin Pharmacol Physiol 2021;34:281-299. doi: 10.1159/000514498 statistical analysis
JASP software version 13.1.
Shapiro-Wilk for normal distribution, Levene test for variance homogeneity
one way ANOVA and Games Howell post hoc tests (parametric data)
Kruskal-Wallis test and Dunn's post hoc tests (non-parametric data)

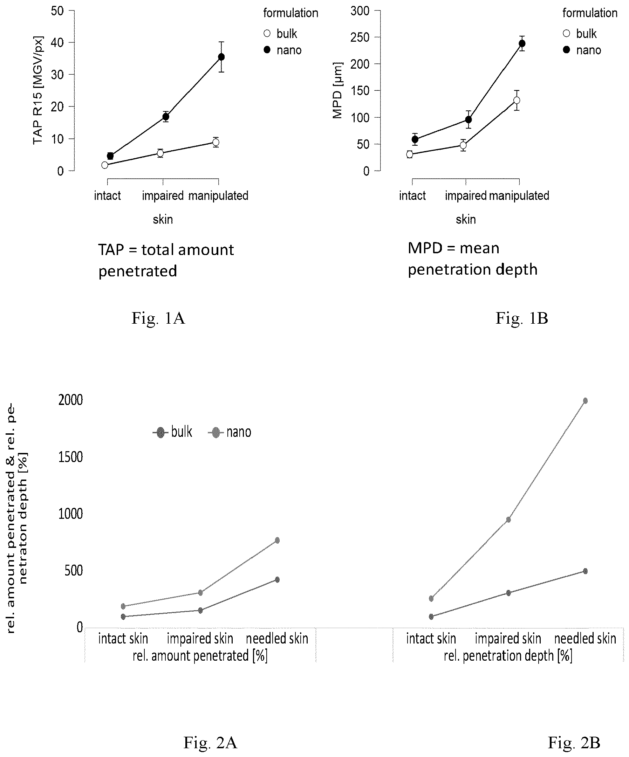

The results are shown in Figure 1A, 1B, 2A and 2B. It can be taken from the figures that the nanocrystals are penetrated into and through the skin in greater amounts and in greater depth compared to bulk material in the differently treated skin materials. In the skin manipulated according to the present invention superior results regarding the penetrated amount and the penetrated depth are obtained compared to intact skin and impaired skin.

## Claims

1. A method for transporting poorly soluble, very poorly soluble and practically insoluble chemical substance formulated as nanoparticles into and through the intact skin, as well as for controlled releasing of said chemical substance formulated as nanoparticles thereafter, wherein said chemical compounds formulated as nanoparticles are applied to the intact skin and treated thereafter with a means for treating the intact skin selected from microneedles, dermaroller and laser so that the chemical compounds formulated as nanoparticles are transported into and through the intact skin.

2. The method according to claim 1, wherein the chemical compound formulated as nanoparticles is selected from nanocrystals, lipidnanoparticles or liposomes.

3. The method according to claim 1 or 2, wherein the chemical compound formulated as nanoparticles have a particle size of less than or equal to 1 000 nm.

4. The method according to any of claims claim 1 to 3, wherein the chemical compound formulated as nanoparticles are suitable for parenteral administration.

5. The method according to any of claims 1 to 4, wherein furthermore a viscosity-changing substance is used, with which a depot in the intact skin is provided, in which the chemical compounds formulated as nanoparticles are embedded so that the releasing of the chemical compound formulated as nanoparticles is controlled.

6. The method according to claim 5, wherein the depot in the intact skin is provided by a gel cushion created in the intact skin, in which the chemical compounds formulated as nanoparticles are embedded.

7. The method according to claim 6, wherein the gel cushion can be prepared beforehand or by applying the chemical compounds formulated as nanoparticles together with a liquid gelling agent to the intact skin and treating both with the means for treating the intact skin so that the chemical compounds formulated as nanoparticles and the liquid gelling agent are transported into and through the intact skin and the liquid gelling agent gels after transport through the intact skin so that the chemical compounds formulated as nanoparticles are embedded therein.

8. The method according to claim 7, wherein the gelling occurs after transport into and through the intact skin due to a change of a pH-value, due to a change in temperature, and/or due to thixotropic properties of the liquid gelling agent.

9. The method according to any of claims 7 or 8, wherein the gelling agent is selected from the group of hyaluronic acids, collagens, dextrans, polyvinylpyrrolidone, cellulose derivatives capable of forming a gel, gummi arabicum, gelatin and poloxamers, in particular poloxamer 407 poloxamer 188, xanthan gum, carrageenan, polyacrylate.

10. The method according to claim 2, wherein the nanocrystal is an active ingredient selected from the group consisting of analgesics, hormones, vaccines, hair growth agents, disinfectants, antiparasitics, fungicides, antibiotics, antifungals, antihistamines, local anaesthetics, bleaching agents and oxidizing agents, antibodies, DNA agents, RNA agents cells, anti-aging, antioxidants, pigments, hair growth agents, anti-acne, hyaluronic acid depots, and disinfectants.

11. Poorly soluble, very poorly soluble and practically insoluble chemical compounds formulated as nanoparticles for use in transporting them into and through the intact skin, as well as for controlled releasing of said chemical compounds formulated as nanoparticles thereafter, wherein said chemical compounds formulated as nanoparticles are applied to the intact skin and treated thereafter with a means for treating the intact skin selected from microneedles, dermarollers and laser so that the chemical compounds formulated as nanoparticles are transported into and through the intact skin.

12. The chemical compounds formulated as nanoparticles according to claim 11, wherein they are used for the treatment of pain, hair loss, hair disease, skin disorders, nail disorders, parasite infections, bacterial infections, fungi infections, treatment of addiction (smoking, THC), hormone disorders (including diabetes), blood pressure adjustment, heart rate adjustment, cancer treatment, treatment of mood, modulation of weight, modulation of appetite, supplementation of vitamins, minerals, trace elements, lung diseases, allergy treatment, prevention of pain, prevention of hair loss, prevention of oxidative stress and diseases/conditions related to oxidative stress. Prevention (including vaccination) of infections (bacteria, virus, fungi, parasites), acne, itching, rosacea, modulation of skin appearance.

13. A method of operating a means for treating the intact skin selected from microneedles, dermaroller and laser for transporting poorly soluble, very poorly soluble and practically insoluble chemical compound formulated as nanoparticles into and through the intact skin, as well as for controlled releasing of said chemical compound formulated as nanoparticles thereafter, wherein said chemical compound formulated as nanoparticles are applied to the intact skin and treated thereafter with said means so that the chemical compound formulated as nanoparticles are transported into and through the intact skin.

14. The use of microneedles, a dermaroller or a laser in a method according to claim 13.
